(19) Europäisches Patentamt
European Patent Office
Office européen des brevets

(11) **EP 4 124 296 A1**

(12) **EUROPEAN PATENT APPLICATION**

(43) Date of publication:
**01.02.2023 Bulletin 2023/05**

(21) Application number: **21382715.7**

(22) Date of filing: **30.07.2021**

(51) International Patent Classification (IPC):
**A61B 5/346** (2021.01)    **A61B 8/02** (2006.01)
**A61B 8/08** (2006.01)

(52) Cooperative Patent Classification (CPC):
**A61B 5/346; A61B 8/02; A61B 8/0883;**
**A61B 8/5284;** A61B 5/361; A61B 8/488

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Koninklijke Philips N.V.**
  **5656 AG Eindhoven (NL)**
• **Centro Nacional de Investigaciones**
  **Cardiovasculares Carlos III (F.S.P.)**
  **28029 Madrid (ES)**

(72) Inventors:
• **Filgueiras Rama, David**
  **28029 Calle de Merchor Almagro, 3. Madrid (ES)**
• **García Quintanilla, Jorge**
  **28029 Calle de Melchor Almagro, 3. Madrid (ES)**
• **Sanchez Gonzalez, Javier**
  **5656 Property & Standards High Tech Campus 5.**
  **AE**
  **Eindhoven (NL)**

(74) Representative: **Philips Intellectual Property &**
**Standards**
**High Tech Campus 52**
**5656 AG Eindhoven (NL)**

(54) **A SYSTEM AND METHOD FOR ASSESSMENT OF ELECTROMECHANICAL REMODELING DURING CARDIAC FIBRILLATION**

(57)    A system is provided for assessing cardiac electromechanical remodeling. It obtains a cardiac electrical activation rate from an ECG system and a cardiac mechanical activation rate from ultrasound data. The sets of data used to derive electrical and mechanical activation rates are preferably obtained simultaneously. An electromechanical relationship is obtained between the electrical and mechanical cardiac activation rates, which is found to be indicative of the cardiac electromechanical remodeling stage.

FIG. 4

**EP 4 124 296 A1**

**Description**

FIELD OF THE INVENTION

The invention relates to a system and method of assessing a subject during cardiac fibrillation, and in particular to assess electromechanical remodeling.

BACKGROUND OF THE INVENTION

Atrial and ventricular fibrillation are serious heart conditions which affect large numbers of the general population. While ventricular fibrillation is a lethal arrhythmia that requires a rapid intervention with direct-current defibrillation to restore a normal heart rhythm, atrial fibrillation only affects the atria, which protects the ventricles from acute failure and imminent cardiac arrest.

Classification of atrial fibrillation (AF) currently relies on temporal criteria from the onset of AF detection and its correlation with any present symptoms. However, about 50% of patients with AF are asymptomatic and AF is detected only incidentally, which leads to a lack of objective clinical information about AF onset. Moreover, despite having the exact episode duration, atrial remodeling progression follows patient-specific patterns which do not allow direct correlation of episode duration with the underlying remodeling stage. This is particularly relevant during the first months of AF progression before overt and severe atrial dilation, when early therapeutic interventions have greater impact on rhythm control and prognosis.

Current guidelines for atrial fibrillation (AF) management from the European Society of Cardiology have identified major knowledge gaps to improve personalized care in patients with AF. The guidelines emphasize a clinical need to improve current assessment of atrial remodeling during AF, which is even more relevant in the context of a global steady increase of AF patients, with an average prevalence of 1.5-2% in developed countries.

Currently, clinical assessment of patient-specific parameters of atrial remodeling associated with AF progression is very limited and requires new clinical alternatives in order to provide physicians with an improved and personalized assessment of the atrial remodeling stage in patients with AF.

Atrial dilatation can be easily assessed with conventional imaging tools (e.g., echocardiogram), but other atrial remodeling parameters such as mechanical remodeling and atrial electrical remodeling are not assessed in regular clinical practice. However, both electrical and mechanical atrial remodeling represent early markers of AF-related atrial changes, which makes their assessment highly relevant to improve current AF characterization and management. Moreover, atrial myopathy may also precede AF onset, establishing a favorable substrate for recurrences and long-term arrhythmia maintenance.

Therefore, novel tools to assess the electromechanical relationship are needed to address current gaps in knowledge and to define the pathological process involved in each AF patient, especially at early disease stages before overt signs of atrial myopathy. Developing reliable and non-invasive clinical tools to assess simultaneous mechanical and electrical parameters during AF is therefore of great interest to obtain a more complete assessment of atrial remodeling stages.

Atrial mechanical activity can for example be assessed using different echocardiography-based tools. However, this task becomes challenging during AF where conventional echocardiography tools such as pulsed Doppler, or more recent tools such as speckle tracking, cannot overcome the intrinsic limitations of irregular wall motion and deformation during AF.

Some preliminary attempts to assess mechanical activity during AF have used tissue velocity imaging at particular atrial wall spots to assess atrial activation rates, for example as disclosed in Limantoro I et al. Heart Rhythm 2014;11:478-84. However, assessing mechanical activity during AF remains a clinical need that requires major methodological improvements and novel signal processing approaches to properly assess mechanical activity in a complex scenario such as during fibrillation (for example AF).

Complementary to atrial mechanical assessment during AF, electrical remodeling is a second parameter associated with early changes in AF progression. Although not implemented in regular clinical practice, electrical remodeling can be evaluated non-invasively using surface ECG tracings after appropriate signal processing to subtract ventricular deflections (QRS-T complexes) and specifically assess an atrial electrical activation rate (hereinafter "EAR") during AF.

US 2019/125311 discloses a monitoring system which combines ultrasonic imaging and an ECG for monitoring a periodic heart movement. The ultrasonic imaging module is used for obtaining an echocardiogram of heart from which mechanical motion identifiers of atriums and ventricles are obtained. The mechanical motion identifiers for atriums and ventricles are output together with ECG information. The system identifies that a patient is in cardiac arrest when there is no contraction but still electrical activity.

Overall, there remains a need for a system and method to provide suitable assessment during fibrillation, to enable cardiac remodeling to be assessed.

SUMMARY OF THE INVENTION

The invention is defined by the claims.

According to examples in accordance with an aspect of the invention, there is provided a system for assessing electromechanical remodeling during cardiac fibrillation, comprising:

> an ECG system for generating an ECG signal;
> an ultrasound imaging system for generating ultrasound data at least for anatomical imaging; and
> a processor adapted to:
>
>> derive movement information from the ultrasound data;
>> derive a mechanical cardiac activation rate during cardiac fibrillation from the movement information;
>> derive an electrical cardiac activation rate during cardiac fibrillation from the ECG signal; and
>> determine an electromechanical relationship between the electrical cardiac activation rate and the counterpart mechanical cardiac activation rate indicative of the electromechanical remodeling stage.

The system is used during cardiac fibrillation; a situation in which periodic systolic and diastolic intervals in the fibrillating cardiac chamber are not present.

This system assesses the relationship between an atrial (or ventricular) EAR (electrical activation rate, as defined above) and a mechanical activation rate (hereinafter "MAR") during cardiac fibrillation, preferably from simultaneously acquired signals, in order to characterize the patient-specific underlying electromechanical remodeling stage. The system uses non-invasive acquisition of recordings of (surface) ECG data and movement analysis of ultrasound to determine an electromechanical relationship during cardiac fibrillation.

The invention uses an assessment of MAR and EAR during fibrillation, preferably simultaneously, to assess the electromechanical relationship between those rates, in particular to determine if the mechanical and electrical rates during fibrillation match each other or diverge. The invention may be implemented with non-invasive simultaneous recordings of surface ECG data and ultrasound to assess a cardiac electromechanical relationship.

As fibrillation-related remodeling evolves, or in the presence of intrinsic atrial or ventricular myopathy, MARs are not able to follow the ECG-based electrical counterpart. This leads to what is defined in this document as electromechanical dissociation (EMD). This means EARs are faster than the preferably simultaneously recorded counterpart MARs.

The inventors have recognized that this EMD is a novel and objective parameter which represents a significant step forward, in particular in assessing atrial remodeling during AF progression, rather than only relying in temporal criteria (which is subjective and often not available) or overt atrial dilation (which is often reflecting advanced remodeling). This parameter thus assists in achieving personalized medicine based on patient-specific parameters of atrial remodeling progression during AF. The invention is however also applicable to the assessment of electromechanical remodeling during ventricular fibrillation.

The invention for example overcomes the well-known limitations of remodeling characterization during early stages of fibrillation progression and provides a highly relevant tool to improve mid-to-long-term prognosis and decision making in the clinic, which represents a significant step forward in the field compared with current clinical methods. These methods for example rely on the existence of overt atrial dilation, which often reflects already advanced stages of atrial remodeling.

The processor may be adapted to derive movement information by applying Tissue Doppler Imaging (TDI). This is a known approach for determining movement of tissues, and it may be derived from the same ultrasound system as used for anatomical imaging.

The electromechanical relationship for example identifies EMD if the electrical cardiac EARs are faster than the cardiac MARs. The EMD may be obtained from one sample myocardial wall region or as a combination from multiple sample wall regions.

The processor may be adapted to derive a parameter representing the level of EMD as a difference between the cardiac EAR and the cardiac MAR. Thus, a level of underlying electromechanical remodeling may be determined.

The system may comprise an ECG system capable of acquiring signals from a single or multiple ECG leads. The system for example uses single lead ECG data and ultrasound data (such as TDI data) to assess the presence of EMD

The single lead is for example the ECG lead II. The atrial signals from this lead are found to provide a good index of the EAR for large atrial areas.

The processor may be adapted to process the ultrasound imaging system data to:

> obtain a 2D anatomical image video;
> segment the myocardial wall from the 2D anatomical image video;

obtain tissue velocity information in respect of the segmented myocardial wall; and

derive the MAR for the myocardial wall.

The myocardial wall is the atrial or ventricular wall. The tissue velocity information is for example derived from an area of the myocardial wall, or from multiple areas.

Atrial or ventricular wall segmentation and tracking is thus used over a time frame (such as around 6 seconds) of 2D image video. The use of 2D video limits the amount of data, although 3D imaging may instead be used. The wall motion tracking and velocity information from motion data over the acquisition period enables the wall MAR to be derived during cardiac fibrillation.

Deriving the cardiac MAR may comprise using a spectral domain method or a time domain method to the motion information, and deriving the cardiac EAR may also comprise using a spectral domain method or a time domain method to the ECG signal.

The spectral domain method may for example determine a dominant frequency (frequency of the highest peak in the spectrum) of the tissue velocity information or ECG signal. A time domain method may for example derive an average cycle length during an acquisition segment.

The system is for example for assessing atrial fibrillation remodeling, and the processor is further adapted to cancel a ventricular tissue velocity component of the obtained tissue velocity information before deriving the cardiac (atrial in this case) MAR.

Thus, for atrial fibrillation monitoring, the ventricular tissue velocity is filtered. The processor may perform the cancelling by using empirical mode decomposition.

Again, for assessing atrial fibrillation remodeling, the processor may be adapted to process the ECG signal to:

remove ventricular components thereby to derive an atrial ECG signal; and

derive an atrial EAR from the atrial ECG signal.

The processor may remove the ventricular components by using principal component analysis (PCA). Thus, PCA may be used for the electrical filtering and empirical mode decomposition may be used for mechanical filtering.

The method may however be applied to electromechanical assessment during ventricular fibrillation. In such a case, atrial components do not need to be filtered in the same way, because electrical or mechanical ventricular signals will not be affected by the small amplitude components of the atrial myocardium.

The invention also provides a method of determining an electromechanical relationship between a cardiac EAR and a cardiac MAR cardiac fibrillation, comprising:

obtaining an ECG signal;

obtaining ultrasound data;

deriving movement information from the ultrasound data;

deriving a cardiac MAR during cardiac fibrillation from the movement information;

deriving a cardiac EAR during cardiac fibrillation from the ECG signal; and

determining the electromechanical relationship between the cardiac EAR and the counterpart cardiac MAR indicative of the electromechanical remodeling stage.

This is the method implemented by the system defined above. It may be used during atrial fibrillation or ventricular fibrillation.

Deriving the cardiac MAR may comprise:

creating a 2D anatomical image video from the ultrasound data;

segmenting the myocardial wall from the 2D anatomical image video;

obtaining tissue velocity information in respect of the myocardial wall; and

deriving the cardiac MAR for the segmented myocardial wall.

The segmenting may identify one or more areas of the myocardial wall to which the velocity assessment is made. The cardiac MAR may thus be derived as a combination of multiple measurements.

The method may comprise identifying EMD if the cardiac EAR or EARs are greater than the cardiac MAR or MARs.

The method may further comprise deriving a parameter representing a level of EMD as a difference between the cardiac EAR or EARs and the cardiac MAR or MARs.

The invention also provides a computer program comprising computer program code means which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a system for assessing cardiac fibrillation remodeling;
Fig. 2 is used to show how the ultrasound data is processed to derive the MAR;
Fig. 3 is used to show how the ECG signal is processed to derive the EAR; and
Fig. 4 shows a method of determining an electromechanical relationship between a cardiac EAR and a cardiac MAR during cardiac fibrillation.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
The invention provides a system for assessing electromechanical remodeling during cardiac fibrillation. It obtains a cardiac EAR from an ECG system and a cardiac MAR from ultrasound data. Both sets of data are preferably acquired simultaneously. An electromechanical relationship is obtained between the EAR and MAR, which is found to be indicative of the electromechanical remodeling stage.
The invention will be described in the context of assessment of an atrial fibrillation patient. However, the concept of the invention may also be applied during ventricular fibrillation.

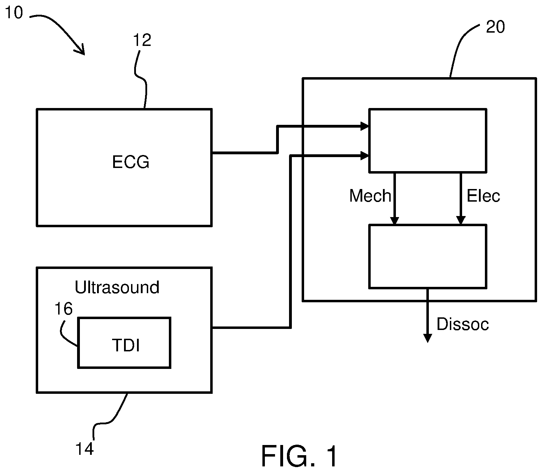

Fig. 1 shows a system 10 for assessing cardiac fibrillation remodeling. The system comprises an ECG system 12 for generating an ECG signal and an ultrasound imaging system 14 for generating ultrasound data at least for anatomical imaging.
In the example shown, the ultrasound system has a TDI functionality 16.
A processor 20 derives movement information from the ultrasound data, and in particular from the TDI data in the example shown. However, movement information may instead be obtained based on image analysis rather than Doppler imaging.
The processor derives a cardiac MAR "Mech" from the movement information and it derives a cardiac EAR "Elec" from the ECG signal.
The processor then determines an electromechanical relationship "Dissoc" between the cardiac EAR and the cardiac MAR. This relationship is indicative of the level of electromechanical remodeling.
This electromechanical relationship in particular identifies (and preferably also quantifies) EMD, during which MARs are not able to follow the ECG-based electrical counterpart. It corresponds to EARs being faster than the preferably simultaneously recorded counterpart MARs.
This EMD parameter represents a significant step forward in assessing cardiac remodeling (atrial remodeling in this example) during early stages of fibrillation progression (AF progression in this example) rather than only relying in temporal criteria or overt atrial dilation, and it can be used to assist the use of personalized medicine. This can improve mid-to-long-term prognosis and decision making in the clinic.
The ECG system 12 records surface ECG tracings. A conventional echocardiography machine can be used for this purpose, and a conventional ultrasound system can be used for the ultrasound imaging, and Doppler imaging if used. The overall system is thus implemented with existing hardware, and enables assessment of early atrial remodeling during AF progression before overt atrial dilation.

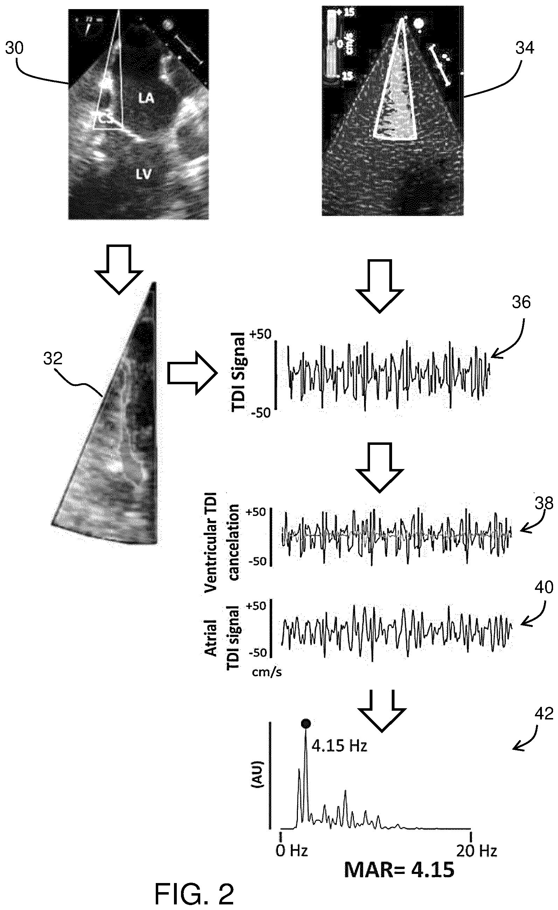

Fig. 2 is used to show how the ultrasound data is processed to derive the MAR.
2D ultrasound images 30 are obtained over an analysis time period (such as six seconds), thus providing a 2D anatomical image video.
The myocardial wall (e.g. atrial wall in this example) is segmented from the 2D anatomical image video to create a segmented image 32. Known segmentation algorithms are used for this purpose. Thus, semi-automatic atrial wall segmentation and automatic tracking may be performed over the time period, and this is for example based on echogenicity criteria on the 2D images. The segmentation may start with the manual selection of the region of interest by the operator by manually demarcating the contour of the atrial wall on an average 2D echocardiography image. Using such a contour as an initial reference, an automatic algorithm can then track the atrial wall motion throughout the cardiac cycles within the acquisition interval.

More specifically, three myocardial areas may be demarcated with high echogenicity (region A, default value: ≥75% of the maximum echogenicity), medium echogenicity (region B, default value: ≥60% of the maximum echogenicity) and low echogenicity (region C, default value: ≥30% of the maximum echogenicity). This is one way to use echogenicity criteria to track the atrial wall over the acquisition interval after the initial step of manual demarcation of the atrial contour.

Automatic but customizable morphological operations (erosion, closing, and removal of small disconnected groups of pixels) may be performed to generate consistent A, B and C regions throughout the whole echocardiography movie.

Using the TDI data 34, tissue velocity information 36 is obtained in respect of the segmented myocardial wall. TDI sequences are obtained using the most optimized beam alignment, image sector angle and image depth enabling imaging of the atrial wall at the maximum frame rate possible (>200 Hz). This enables specific analysis of TDI signals from the segmented atrial wall within the image section angle during acquisition.

Local TDI signals from each pixel within the segmented atrial wall may be averaged to generate higher quality signals over the measurement period.

The tissue velocity information 36 is raw TDI data. This raw TDI data is further processed to obtain the atrial MARs.

For example, empirical mode decomposition is used to minimize the variable ventricular motion effects on atrial TDI signals. This uses the original Doppler signals in the time domain to obtain intrinsic oscillatory modes without assuming periodicity. This enables subtraction of mechanical artefacts produced by ventricular contractions from the averaged TDI signals. This results in a signal 38 for cancelling the ventricular TDI signal so that the atrial TDI signal 40 can be obtained. This method is thus effective even in the presence of time-varying ventricular motion artifacts due to irregular diastolic intervals in AF.

A TDI-derived MAR is then calculated using spectral analysis and the fast Fourier transform to convert the tracing into the frequency domain (power spectral density) as shown by plot 42. The frequency of the highest peak in the power spectral density (i.e. dominant frequency) may be used to obtain an objective and rapid calculation of the average MAR, shown a 4.15 Hz in this example.

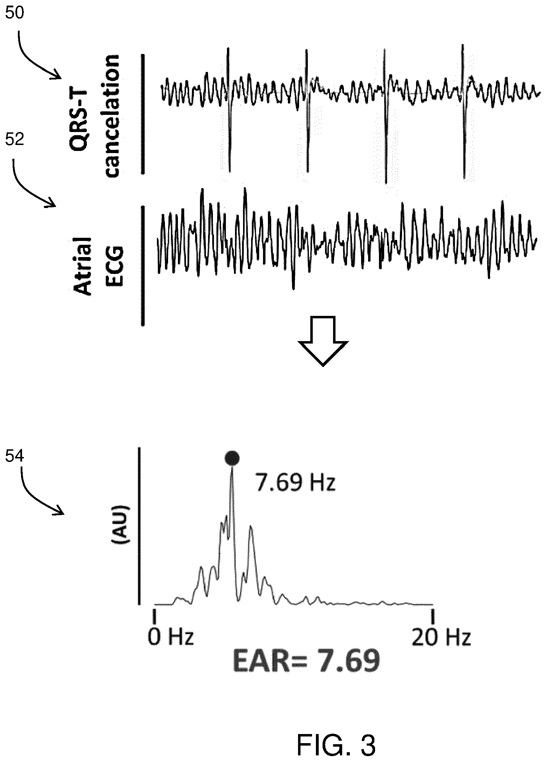

Fig. 3 is used to show how the ECG signal is processed to derive the EAR. The ECG signal is for example from the ECG lead II. It has been found that signals from lead II encompass the activation rate of large endocardial atrial areas. This minimizes the chance of TDI acquisition on atrial areas electrically activating much faster or slower than the dominant frequency peak detected on lead II.

Preferably simultaneous atrial electrical activity is obtained by removing ventricular components (QRS-T complexes) by generating a QRST-T cancellation signal 50, so that the atrial ECG signal 52 can be obtained.

To estimate the cancellation signal 50 to be subtracted from the raw ECG signal, Principal Component Analysis may be used.

An ECG-derived EAR is then calculated using spectral analysis and the fast Fourier transform to convert the atrial ECG signal into the frequency domain (power spectral density) as shown by plot 54. The frequency of the highest peak in the power spectral density (i.e. dominant frequency) may again be used to obtain an objective and rapid calculation of the average atrial EAR, shown as 7.69 Hz in this example.

It is noted that spectral analysis to obtain a frequency peak is only one example of how to obtain a representative rate. More generally, a spectral domain method or a time domain method may be applied to the motion information (i.e. the TDI data in this example) and the ECG data, and deriving the cardiac EAR may also comprise using a spectral domain method or a time domain method to the ECG signal.

The spectral domain method may for example determine a dominant frequency of the tissue velocity information or ECG signal as mentioned above. A time domain method may for example instead derive an average cycle length during an acquisition segment.

The analysis of the motion data (TDI data) and the ECG signal is for example based on a six second data stream (with frame rate >200 Hz using either transthoracic or transesophageal echocardiography transducers). To ensure accurate feature extraction the six second segment is acquired independently of the patient heart rate.

The data from the TDI sequences and the ECG system are for example processed in the echocardiography machine or alternatively exported in DICOM format for external processing to obtain:

    i) Six second 2D echocardiography movies,
    ii) Tissue velocity information from TDI data for the specific atrial region or regions, and
    iii) Preferably simultaneous lead II ECG signals.

An operator of the system is for example able to visualize both the echocardiography movie and TDI data on a superimposed layer at a graphical user interface.

The presence of atrial EMD is defined as occurring when there is a faster atrial EAR than the preferably simultaneous counterpart TDI-derived MAR (EAR>MAR). In that case, EMD is quantified as:

$$EMD=EAR-MAR$$

A minimum of one segmented sample region from the myocardial wall (atria) is required, with preferably simultaneous acquisition of ECG and motion detection (e.g. TDI imaging).

However, multiple locations may be segmented, such as the left and right atrial wall. In such a case, the EMD may be quantified as the average between the two locations:

$$EMD_{index} = \frac{EMD_{right\ atrium\ (RA)} + EMD_{left\ atrium\ (LA)}}{2} = \frac{(EAR - MAR_{RA}) + (EAR - MAR_{LA})}{2}$$

Using the same approach, a higher number of sample regions from the right or left atrium can also be included to enable more accurate assessment of the electromechanical relationship.

The methodology can be implemented in current echocardiography machines after software and graphical user interface implementation. This methodological approach provides non-invasive characterization of the mechanical and electrical atrial remodeling stage, for example in patients with AF before overt atrial dilation. This is not available in current clinical practice. Moreover, atrial remodeling characterization has been identified as an area of further improvement in current AF management, since the atrial remodeling stage has prognosis impact.

Moreover, early identification of atrial remodeling progression during AF enables the implementation of earlier, and therefore more effective, rhythm control strategies. This will therefore impact in clinical practice in the large population of patients with AF undergoing echocardiography studies. Echocardiography studies can then provide clinically-relevant information about their atrial remodeling stage to improve characterization of their own AF substrate in order to further improve clinical management to achieve a more personalized patient care.

The discussion above is based on the use of the system for assessment during atrial fibrillation. However, the same approach may be used in the ventricle for ventricular fibrillation. Thus, the invention applies more generally to cardiac fibrillation.

In the case of ventricular fibrillation, atrial activation components do not need to be filtered in the same way as described above for the filtering of ventricular components in atrial fibrillation assessment. Electrical and mechanical ventricular signals during ventricular fibrillation will not be affected by the small amplitude components of the atrial myocardium.

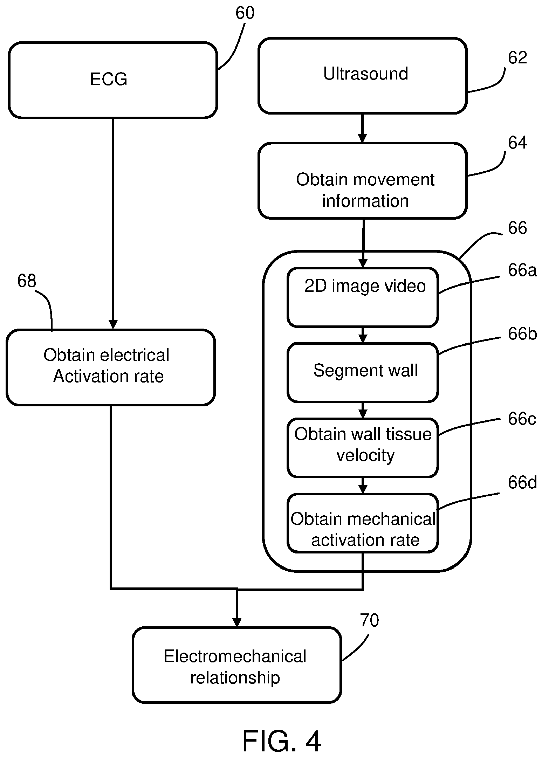

Fig. 4 shows a method of determining an electromechanical relationship between a cardiac EAR and a cardiac MAR during cardiac fibrillation. The method comprises obtaining an ECG signal in step 60 and obtaining ultrasound data in step 62. These signals are preferably obtained simultaneously.

In step 64, movement information is obtained from the ultrasound data, for example by extracting TDI data.

In step 66 a cardiac MAR is obtained during the cardiac fibrillation. This involves:

creating a 2D anatomical image video from the ultrasound data in step 66a;
segmenting the myocardial wall from the 2D anatomical image video in step 66b;
obtaining tissue velocity information in respect of the myocardial wall in step 66c; and
deriving the cardiac MAR for the segmented myocardial wall in step 66d.

A cardiac EAR during cardiac fibrillation is obtained in step 68 from the ECG signal. The cardiac EAR and the MAR that are measured are counterparts that are preferably simultaneous.

An electromechanical relationship (e.g. level of EMD) between the EAR and the preferably simultaneous MAR is obtained in step 70. This relationship is indicative of cardiac electromechanical remodeling.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to".

Any reference signs in the claims should not be construed as limiting the scope.

**Claims**

1. A system (10) for assessing electromechanical remodeling during cardiac fibrillation, comprising:

   an ECG system (12) for generating an ECG signal;
   an ultrasound imaging system (14) for generating ultrasound data at least for anatomical imaging; and
   a processor (20) adapted to:

   derive movement information from the ultrasound data;
   derive a mechanical cardiac activation rate (Mech) during cardiac fibrillation from the movement information;
   derive an electrical cardiac activation rate (Elec) during cardiac fibrillation from the ECG signal; and
   determine an electromechanical relationship (Dissoc) between the electrical cardiac activation rate and the counterpart mechanical cardiac activation rate indicative of the electromechanical remodeling stage.

2. The system of claim 1, wherein the processor (20) is adapted to derive movement information by applying Tissue Doppler Imaging.

3. The system of claim 1 or 2, wherein the electromechanical relationship (Dissoc) identifies electromechanical dissociation if the electrical cardiac activation rate or rates are greater than the mechanical cardiac activation rate or rates

4. The system of claim 3, wherein the processor (20) is adapted to derive a parameter representing the level of electromechanical dissociation as a difference between the electrical cardiac activation rate and the mechanical cardiac activation rate.

5. The system of any one of claims 1 to 4, comprising a single or multiple lead ECG system.

6. The system of claim 5, wherein the ECG system is a single lead system with the ECG lead II.

7. The system of any one of claims 1 to 6, wherein the processor (20) is adapted to process the ultrasound imaging system data to:

   obtain a 2D anatomical image video (30);
   segment the myocardial wall from the 2D anatomical image video;
   obtain tissue velocity information in respect of the segmented myocardial wall (32);
   and derive the mechanical activation rate for the myocardial wall.

8. The system of any one of claims 1 to 7, wherein:

   deriving the mechanical cardiac activation rate comprises using a spectral domain method or a time domain method to the motion information; and
deriving the electrical cardiac activation rate comprises using a spectral domain method or a time domain method to the ECG signal.

9. The system of any one of claims 1 to 8, for assessing atrial fibrillation remodeling, wherein the processor is further adapted to cancel a ventricular tissue velocity component of the obtained tissue velocity information before deriving the mechanical cardiac activation rate.

10. The system of any one of claims 1 to 9, for assessing atrial fibrillation remodeling, wherein the processor is adapted to process the ECG signal to:

remove ventricular components thereby to derive an atrial ECG signal; and
derive an electrical atrial activation rate from the atrial ECG signal.

11. A method of determining an electromechanical relationship between an electrical cardiac activation rate and a mechanical cardiac activation rate during cardiac fibrillation, comprising:

(60) obtaining an ECG signal;
(62) obtaining ultrasound data;
(64) deriving movement information from the ultrasound data;
(66) deriving a mechanical cardiac activation rate during cardiac fibrillation from the movement information;
(68) deriving an electrical cardiac activation rate during cardiac fibrillation from the ECG signal; and
(70) determining the electromechanical relationship between the electrical cardiac activation rate and the counterpart mechanical cardiac activation rate indicative of the electromechanical remodeling stage.

12. The method claim 11, wherein the deriving the mechanical cardiac activation rate comprises:

(66a) creating a 2D anatomical image video from the ultrasound data;
(66b) segmenting the myocardial wall from the 2D anatomical image video;
(66c) obtaining tissue velocity information in respect of the myocardial wall; and
(66d) deriving the mechanical cardiac activation rate for the segmented myocardial wall.

13. The method of claim 11 or 12, comprising identifying electromechanical dissociation if the electrical cardiac activation rate or rates are greater than the mechanical cardiac activation rate or rates.

14. The method program of claims 13, wherein the method further comprises deriving a parameter representing a level of electromechanical dissociation as a difference between the electrical cardiac activation rate and the mechanical cardiac activation rate.

15. A computer program comprising computer program code means which is adapted, when said program is run on a computer, to implement the method of any one of claims 11 to 14.

FIG. 1

FIG. 2

50 QRS-T cancelation

52 Atrial ECG

54 (AU)

7.69 Hz

0 Hz    20 Hz

EAR= 7.69

FIG. 3

FIG. 4

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2019/125311 A1 (ZHANG XIAOCUI [CN] ET AL) 2 May 2019 (2019-05-02) <br> * paragraphs [0001] – [0009] * <br> * paragraphs [0035] – [0037] * <br> * paragraphs [0048] – [0049] * <br> ----- | 1-15 | INV. <br> A61B5/346 <br> A61B8/02 <br> A61B8/08 |
| X | US 7 130 681 B2 (MEDTRONIC INC [US]) 31 October 2006 (2006-10-31) <br> * page 1, lines 1-26 * <br> * page 3, line 55 – page 4, line 37 * <br> * page 11, line 30 – page 13, line 65 * <br> ----- | 1-5, 8-13,15 | |
| A | US 2009/306732 A1 (ROSENBERG STUART [US] ET AL) 10 December 2009 (2009-12-10) <br> * paragraphs [0065] – [0070] * <br> * paragraphs [0125] – [0165] * <br> ----- | 1-15 | |
| A | US 2014/088943 A1 (TRAYANOVA NATALIA A [US] ET AL) 27 March 2014 (2014-03-27) <br> * paragraphs [0079] – [0095] * <br> ----- | 1-15 | TECHNICAL FIELDS SEARCHED (IPC) <br><br> A61B |

The present search report has been drawn up for all claims

| Place of search | Date of completion of the search | Examiner |
|---|---|---|
| The Hague | 14 January 2022 | Loveniers, Kris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                     .......
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 38 2715

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2022

| Patent document cited in search report | | Publication date | Patent family member(s) | | Publication date |
|---|---|---|---|---|---|
| US 2019125311 | A1 | 02-05-2019 | CN | 107708570 A | 16-02-2018 |
| | | | CN | 112043259 A | 08-12-2020 |
| | | | US | 2019125311 A1 | 02-05-2019 |
| | | | WO | 2017008202 A1 | 19-01-2017 |
| US 7130681 | B2 | 31-10-2006 | CA | 2525378 A1 | 25-11-2004 |
| | | | DE | 602004007789 T2 | 30-04-2008 |
| | | | EP | 1622679 A1 | 08-02-2006 |
| | | | JP | 4477638 B2 | 09-06-2010 |
| | | | JP | 2007503280 A | 22-02-2007 |
| | | | US | 2004225332 A1 | 11-11-2004 |
| | | | WO | 2004101066 A1 | 25-11-2004 |
| US 2009306732 | A1 | 10-12-2009 | US | 2009306732 A1 | 10-12-2009 |
| | | | US | 2011295137 A1 | 01-12-2011 |
| US 2014088943 | A1 | 27-03-2014 | AU | 2012214163 A1 | 29-08-2013 |
| | | | AU | 2016204898 A1 | 04-08-2016 |
| | | | CA | 2827042 A1 | 16-08-2012 |
| | | | EP | 2672889 A2 | 18-12-2013 |
| | | | IL | 227906 A | 29-12-2016 |
| | | | JP | 6203641 B2 | 27-09-2017 |
| | | | JP | 2014512201 A | 22-05-2014 |
| | | | US | 2014088943 A1 | 27-03-2014 |
| | | | US | 2020375487 A1 | 03-12-2020 |
| | | | WO | 2012109618 A2 | 16-08-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019125311 A

**Non-patent literature cited in the description**

- **LIMANTORO I et al.** *Heart Rhythm,* 2014, vol. 11, 478-84